Europäisches Patentamt

European Patent Office

Office européen des brevets

(11) Veröffentlichungsnummer: **0 498 160 A2**

# EUROPÄISCHE PATENTANMELDUNG

(21) Anmeldenummer: **92100326.5**

(51) Int. Cl.5: **A61L 2/26**

(22) Anmeldetag: **10.01.92**

(30) Priorität: **02.02.91 DE 4103146**

(43) Veröffentlichungstag der Anmeldung:
**12.08.92 Patentblatt 92/33**

(84) Benannte Vertragsstaaten:
**AT BE DE FR GB IT**

(71) Anmelder: **Richard Wolf GmbH**
**Pforzheimer Strasse 32**
**W-7134 Knittlingen(DE)**

(72) Erfinder: **Mönch, Harry**
**Schwarbstrasse 4**
**W-7134 Knittlingen(DE)**

(74) Vertreter: **Vollmann, Heiko, Dipl.-Ing. et al**
**Patentanwälte Wilcken, H., Dr. Wilcken, Th.,**
**Dipl.-Ing. Vollmann, H., Dipl.-Ing.,**
**Musterbahn 1**
**W-2400 Lübeck 1(DE)**

(54) **Instrumenten-Sterilisationsbehälter.**

(57) Es ist ein Behälter zur Aufnahme von vorzugsweise medizinischen Instrumenten und/oder Teilen derselben beschrieben. Der Behälter dient der Aufbewahrung, Desinfektion oer Sterilisation dieser Instrumente und besteht aus einer ersten Wanne (1), in die eine zweite Wanne (2) einsetzbar ist. Die ineinandergestellten Wannen sind mit einem Behälterdeckel (3) lösbar abdeckbar und zu einer Behältereinheit verbindbar. Die Instrumente lagern auf flexiblen Noppenmatten (14,19), die auf den Bodenflächen (17,18) der Wannen (1,2) und an der Deckelfläche punkt- oder linienförmig gestützt aufliegen. Die Matten (14) sind lösbar befestigt. Es können weitere Noppenmatten (14,19) zum zusätzlichen Fixieren der eingelegten Instrumente vorgesehen sein.

FIG. 1

EP 0 498 160 A2

Die Erfindung geht aus von einem Behälter zur Aufnahme von medizinischen Instrumenten und/oder Teilen derselben, insbesondere zum Zwecke des Versandes, der Aufbewahrung, Desinfektion und/oder Sterilisation, bestehend aus einer Wanne mit einem darin befindlichen Einsatz sowie einem die Wanne mit dem Einsatz abdeckenden lösbaren Behälterdeckel.

Behälter dieser Art sind in mannigfachen Ausführungen bekannt.

So ist aus der DE-P 34 14 679 ein aus Kunststoff tiefgezogener Behälter mit einem durch einen Deckel verschließbaren, mit Löchern in seinem Boden versehenen Wannenteil zu entnehmen. Der Behälter dient speziell der Aufnahme eines Ultraschallwandlers, zu welchem Zweck der Wannenteil mit entsprechend gestalteten Vorsprüngen versehen ist. Deckel und Wannenteil sind durch federnde Klemmlaschen lösbar miteinander verbindbar.

Aus der DE-P 34 38 878 ist eine Vorrichtung zur Desinfektion von Endoskopen und Zubehör bekannt, die aus einer die Desinfektionsflüssigkeit aufnehmenden Wanne besteht, in die ein Siebkorb mit dem zu desinfizierenden Teilen eingetaucht werden kann. Nach der Desinfektion kann der Siebkorb herausgehoben und nach axialer Verschiebung durch in der Wanne vorgesehener seitlicher Vorsprünge abgestützt werden, so daß die Desinfektionsflüssigkeit abtropfen kann.

Die DE-P 39 18 147 zeigt einen Sterilisationsbehälter zur Aufnahme von Endoskopen oder Teilen davon zum Zwecke der Sterilisation oder Desinfektion. Der Behälter besteht aus einer Drahtkonstruktion und weist ein Unterteil mit Halterungen für die zu behandelnden Teile sowie ein lösbar auf dem Unterteil festlegbares Oberteil mit nach innen gerichteten Stützelementen auf. Der Behälter wird zur Behandlung der in diesem befindlichen Teile in eine entsprechende Apparatur eingestellt.

Schließlich ist aus der DE-P 34 36 489 ein Behälter entnehmbar, der für den Versand, die Desinfektion, Sterilisation und Lagerung von unterschiedlichen Instrumenten ausgebildet ist. Die Behälterwanne ist durch einen Deckel losbar verschließbar und in beide Teile sind auswählbare Stützteile einsetzbar, die ein oder mehrere Instrumente nebeneinander im Behälter abstützen.

Während diese bekannten Behälter sowohl hinsichtlich der Aufnahme und Halterung der Instrumente als auch zur Durchführung der Behandlung gut geeignet sind, ist ihre Fertigung zum Teil problematisch mit dem Effekt hoher Gestehungskosten. Zudem ist bei den bekannten Ausführungen nachteilig, daß jeweils ein Instrumentensatz einem bestimmten Behälter zugeordnet wird, wobei der zur Verfügung stehende Raum nicht vollständig genutzt wird, so daß für zusätzliche Instrumente zusätzliche Behälter benötigt werden und daher der Raumbedarf erhöht wird. Im Falle der Verwendung von komplementär zum Instrument ausgebildeten, flexibel verformbaren Aufnahmen, ergibt sich schließlich noch der Nachteil, daß die Auslüftung erheblich behindert wird. Gleichzeitig steht dem Vorteil einer guten Ausnutzbarkeit des zur Verfügung stehenden Raumes in einem Behälter durch Einlegen einer Vielzahl von Instrumenten bzw. Instrumententeilen der Nachteil gegenüber, daß die Teile nicht in ihrer Lage fixiert sind, wodurch bei unsachgemäßer Handhabung Beschädigungen an den Instrumenten möglich sind.

Es ist daher die Aufgabe der vorliegenden Erfindung, einen Behälter vorzuschlagen, der in der Lage ist, eine Vielzahl von einzelnen Instrumenten und/oder Teilen derselben gemeinsam lagegesichert aufzunehmen und einer Desinfektion oder Sterilisaton zu unterziehen, wobei Flüssigkeitsrückstände aufgrund ausreichender Durchlüftung sicher vermieden werden. Darüberhinaus soll dieser Behälter billiger herstellbar, universell verwendbar, mit anderen Behältern kombinierbar, stapelbar und auf einfache Weise sicher verschließbar sein.

Diese Aufgabe ist erfindungsgemäß durch einen Behälter der einleitend angeführten Art dadurch gelöst, daß der Einsatz als Wanne ausgebildet ist, an den Bodenflächen sowohl der ersten Wanne als auch der zweiten Wanne aufragende Noppen aufweisende Noppenmatten, vorzugsweise aus Silikon, lösbar angebracht sind und an der Innenseite des Behälterdeckels an seiner Innenseite zumindest partiell Noppenmatten festlegbar sind, deren Noppen gegen die Bodenflächen gerichtet sind.

Damit lassen sich die zu behandelnden Instrumente auf zwei Ebenen festlegbar und sicher unterbringen, wobei die Fixierung in der unteren Ebene bedarfsweise noch dadurch verbessert werden kann, daß zusätzlich eine Noppenmatte auf die dort befindlichen Teile gelegt erreicht wird, wodurch eine sehr gute Desinfektion und/oder Sterilisation wird. Des weiteren ist der erfindungsgemäße Behälter billiger herstellbar, universell verwendbar, mit anderen Behältern kombinierbar, stapelbar und auf einfache Weise sicher verschließbar.

Bevorzugte Modifikationen des erfindungsgemäßen Behälters sind den Unteransprüchen zu entnehmen.

Die Erfindung ist nachstehend anhand eines in der Zeichnung dargestellten Ausführungsbeispiels näher erläutert. Es zeigen:

Figur 1    eine Seitenansicht des Behälters, zur Hälfte längsgeschnitten dargestellt,

Figur 2    eine teilweise Draufsicht auf den Behälter mit partiell ausgebrochenem Deckel,

Figur 3    eine vergrößerte Teilschnittdarstellung des Deckelverschlusses,

Figur 4 ein Ausführungsbeispiel für die Halterung der Noppenmatten in dem Deckel,

Figur 5 ein anderes Ausführungsbeispiel für eine solche Halterung,

Figur 6 eine vergrößerte Teilschnittdarstellung einer Druckknopfverbindung für die Festlegung der Noppenmatte.

Der erfindungsgemäße Behälter umfaßt eine erste Wanne 1 in etwa quaderförmiger Gestalt, eine zweite, in die erste Wanne 1 einsetzbare Wanne 2 sowie einen Behälterdeckel 3. Beide Wannen 1 und 2 sind an ihrer offenen Seite mit einem U-förmig profilierten umlaufenden Rand 4 bzw. 5 versehen, wobei die Profilierung des Randes 5 der zweiten Wanne 2 demjenigen der ersten Wanne 1 umlaufend übergreift. Der Behälterdeckel 3 ist mit einem Rand 6 versteift, der seinerseits den umlaufenden Rand 5 der zweiten Wanne 2 übergreifen kann. Der Behälterdeckel 3 weist weiter jeweils an beiden Stirnseiten zwei zueinander beabstandete Ausformungen 7 mit Taschen 8 für die Aufnahme eines Lagerstiftes 9 auf. Der Lagerstift 9 dient einem federelastischen Klammerelement 10 als Lagerung, welches U-förmig ausgebildet ist und in Verriegelungsstellung die beiden Wannen 1 und 2 an ihrem Randbereich umgreift. Aufgrund der federelastischen Ausbildung sind dabei alle Behälterteile 1,2 und 3 unter Federvorspannung miteinander lösbar verbunden. Um die Lagerstifte 9 gegen Herausfallen zu sichern, können Verschlußteile 11 vorgesehen sein, die die Taschen 8 nach der Montage des Klammermechanismus abdecken und die auf geeignete Weise durch Rastung festlegbar sind.

Der Behälterdeckel 3 ist stellenweise mit eingezogenen Feldern 12 versehen, die mit nach innen weisenden punkt- oder linienförmigen Erhöhungen 13 versehen sind (Figuren 4,5 und 6). Sie stützen in die Felder 12 eingelegte Noppenmatten 14, die dadurch gegenüber der Deckelfläche freigestellt gehalten werden. Jede Noppenmatte 14 ist mit Noppen 15 bestückt und wird an dem Behälterdeckel 3 jeweils durch ein Fixierelement 16 festgelegt, das derart ausgestaltet ist, daß es mit entsprechenden An- oder Ausformungen der Noppenmatte 14 eine lösbare Druckknopfverbindung bildet (Figuren 4, 5 und 6). Es kann auch die gesamte Deckelfläche mit einer Noppenmatte 14 versehen sein.

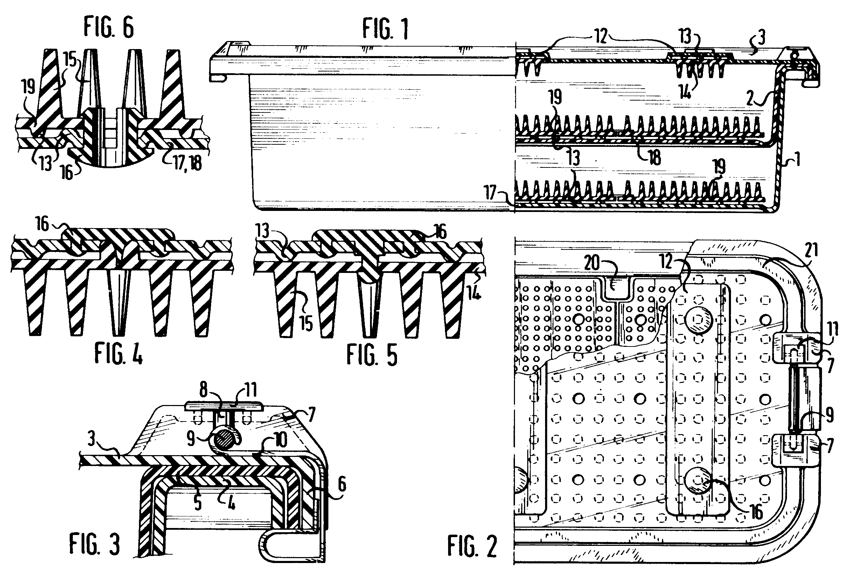

Die Wannen 1 und 2 sind ebenfalls an ihren Bodenflächen 17 bzw. 18 wie der Behälterdeckel 3 im Bereich der Felder 12 mit punkt- oder linienförmigen Erhöhungen 13 versehen (Figur 1).Sie dienen der Abstützung von weiteren Noppenmatten 19, die durch seitliche Ausformungen 20 an ihrer Position fixierbar sind.

Alternativ oder zusätzlich können die Erhöhungen 13 auch an den vorzugsweise aus Silikon bestehenden Matten 14, 19 vorgesehen sein, um die Auslüftung und Trocknung zu verbessern.

Die zu behandelnden Instrumente werden auf die Noppenmatten 19 in den vereinzelten Wannen 1 und 2 aufgelegt, wobei sie im Bereich ihrer Auflagenfläche die Noppen 15 verdrängen und durch die außerhalb der Auflagefläche befindlichen, unverformt bleibenden Noppen seitlich fixiert werden. Bedarfsweise können nun noch weitere, lose Noppenmatten auf die in die Wanne 1 eingebrachten Instrumente mit ihren Noppen zu den Instrumenten weisend aufgelegt werden, wonach die Wanne 2 in die erste Wanne 1 eingehängt wird. Dabei werden die frei aufliegenden Noppenmatten durch die Bodenfläche 17 der Wanne 2 gegen die Instrumente gedrückt, so daß diese auch von oben zwischen den Noppen 15 der unteren Matte 19 eingebettet werden. Das gleiche geschieht mit den in der zweiten Wanne 2 eingelagerten Instrumenten mittels der an dem Behälterdeckel 3 befestigten Noppenmatten 14, wenn dieser abschließend über die ineinander gefügten Wannen 1 ud 2 gestülpt und mittels der Klammerelemente 10 zu einer Behältereinheit verbunden wird.

Um eine Stapelung der Behälter zu ermöglichen, kann der Behälterdeckel 3 mit einem Wulstrand 21 versehen sein, der die äußere Deckelfläche umgibt, die der Bodenfläche 17 der Wanne 1 entspricht.

Die punkt- oder linienförmige Stützung der die Instrumente aufnehmenden Noppenmatten sowie die Einlagerung der Instrumente in ein Noppenfeld gewährleisten eine ausreichende Durchlüftung und damit die Vermeidung von Flüssigkeitsrückständen nach der Behandlung.

**Patentansprüche**

1. Behälter zur Aufnahme von medizinischen Instrumenten und/oder Teilen derselben, zum Zwecke des Versandes, der Aufbewahrung, Desinfektion und/oder Sterilisation, bestehend aus einer Wanne mit einem darin befindlichen Einsatz sowie einem die Wanne mit dem Einsatz abdeckenden lösbaren Behälterdeckel, dadurch gekennzeichnet, daß der Einsatz als Wanne (2) ausgebildet ist, an den Bodenflächen (17 bzw. 18) sowohl der ersten Wanne (1) als auch der zweiten Wanne (2) aufragende Noppen (15) aufweisende Noppenmatten (19) lösbar angebracht sind und an der Innenseite des Behälterdeckels (3) zumindest partiell Noppenmatten (14) festlegbar sind, deren Noppen (15) gegen die Bodenflächen (17 und 18) gerichtet sind.

2. Behälter nach Anspruch 1, dadurch gekennzeichnet, daß die Bodenflächen (17 und 18) der Wannen (1, 2) und/oder die Noppenmatten (14 bzs. 19) mit punkt- oder linienförmigen abstützenden Erhöhungen (13) ausgestattet sind.

3. Behälter nach Anspruch 1 oder 2, dadurch gekennzeichnet, daß die Noppenmatten (14) des Behälterdeckels (3) mittels Fixierelementen (16) festlegbar sind.

4. Behälter nach einem der Ansprüche 1 bis 3, dadurch gekennzeichnet, daß die beiden Wannen (1 und 2) jeweils einen U-förmig profilierten, umlaufenden Rand (4 bzw. 5) aufweisen, wobei die Profilierung derartig ist, daß der Rand (5) der zweiten Wanne (2) denjenigen (4) der ersten Wanne (1) umlaufend übergreift.

5. Behälter nach einem der Ansprüche 1 bis 4, dadurch gekennzeichnet, daß der Behälterdeckel (3) mit einem die profilierten Ränder (4 und 5) gemeinsam übergreifenden Rand (6) versehen ist.

6. Behälter nach einem der Ansprüche 1 bis 5, dadurch gekennzeichnet, daß der Behälterdeckel (3) mit federelastisch ausgebildeten Klammerlementen (10) versehen ist, die dessen Fixierung auf mindestens einer der Wannen (1 bzw. 2) durch Umgreifen des entsprechenden U-förmigen Randes (4 bzw. 5) ermöglicht.

7. Behälter nach Anspruch 6, dadurch gekennzeichnet, daß die Klammerelemente (10) in oder außer Eingriff schwenkbar angeordnet sind.

8. Behälter nach einem der Ansprüche 1 bis 7, dadurch gekennzeichnet, daß der Behälterdeckel (3) einen umlaufenden, das Stapeln gleicher Behälter ermöglichenden Wulstrand (21) aufweist.

FIG. 1

FIG. 2

FIG. 3

FIG. 4

FIG. 5

FIG. 6